# DEMANDE DE BREVET EUROPEEN

(11) **EP 4 656 135 A1**
(43) Date de publication de la demande: **03.12.2025**
(21) Numéro de dépôt: 25177764.5
(22) Date de dépôt: 20.05.2025
(51) Int. Cl.: A61B 5/20, A61B 5/00, A61B 10/00, G01N 33/493, A61B 5/145

(54) **BRAS DE FIXATION POUR DISPOSITIF D'ANALYSE D'URINE**

(30) Priorité: 30.05.2024 FR 2405614
(71) Demandeur: Withings, 92130 Issy-Les-Moulineaux (FR)
(72) Inventeur: Pons, Arthur, 92130 Issy-les-Moulineaux (FR)
(74) Mandataire: Withings IP

(57) **Abrégé**

L'invention concerne un ensemble pour analyse d'urine comprenant :
- un dispositif d'analyse d'urine (100) configuré pour être placé entièrement dans une cuvette (106) de toilettes et comprenant un boîtier (204) et un élément d'attache (510) disposé sur une face arrière (222) du boîtier,
- un bras de fixation (600) configuré pour positionner le dispositif d'analyse d'urine entièrement dans la cuvette (106) de toilettes, le bras de fixation (600) comprenant une portion d'attache proximale configurée pour coopérer avec l'élément d'attache (510) du dispositif d'analyse d'urine; la portion d'attache proximale formant un espace de réception pour le boîtier (204), dans lequel l'espace de réception s'étend depuis la face arrière (222) jusqu'au-delà d'un bord (550) de la face arrière.

## Description

La présente invention concerne un ensemble pour analyse d'urine comprenant un dispositif d'analyse d'urine. Le dispositif d'analyse d'urine comprend un boîtier configuré pour être placé entièrement dans une cuvette de toilettes. L'ensemble comprend en outre un bras de fixation pour fixer le dispositif d'analyse d'urine à la cuvette des toilettes.

### Etat de la technique

De nombreux paramètres biologiques sont identifiables dans l'urine d'un individu. Par exemple, des problèmes de santé tels qu'une infection des voies urinaires, un diabète ou une insuffisance rénale peuvent être détectés à partir d'un échantillon d'urine. L'échantillon d'urine peut également refléter la qualité d'un régime alimentaire, identifier une période de fertilité ou de grossesse et détecter la consommation de drogues ou de tabac. Il est donc intéressant de surveiller périodiquement divers paramètres biologiques.

Il est connu de proposer des dispositifs de toilettes avec une fonction d'analyse d'urine. Ces dispositifs sont capables de prélever des échantillons d'urine dans les toilettes et de les analyser pour déterminer le niveau d'un paramètre biologique.

Un tel dispositif d'analyse d'urine est notamment décrit dans le document WO2021/175909. Ce document présente un dispositif pour l'analyse d'urine. Le dispositif se loge dans les toilettes et recueille un échantillon d'urine avant d'effectuer une analyse optique sur un support de test. Le dispositif comprend une station et une cartouche, également appelée support rotatif, qui peut être retirée et remplacée de la station. La cartouche contient des bandelettes urinaires, c'est-à-dire des bandelettes enduites ou imprégnées d'un réactif réagissant avec l'urine.

Afin de placer un tel dispositif d'analyse d'urine dans les toilettes, plusieurs solutions ont été proposées. Le document WO2021/175909 propose par exemple une ventouse sur la face arrière du boîtier du dispositif destinée à coopérer avec la paroi des toilettes (paragraphe 106) ou un crochet monté sur un rebord de la cuvette des toilettes (paragraphe 110). Le document WO2023/036805 suggère des aimants ou une surface adhésive à l'arrière du boîtier pour coopérer avec la paroi interne des toilettes (paragraphe 93). Au *Consumer Electronics Show* (CES) de Las Vegas en janvier 2023, un dispositif d'analyse d'urine avec un crochet a été présenté. Le dispositif d'analyse d'urine comprend un boîtier avec une face arrière, orientée vers une paroi de la cuvette de toilette. Sur la face arrière, un ergot est positionné ; cet ergot coopère avec un orifice d'un bras, qui s'accroche ensuite avec un crochet au rebord de la cuvette. Entre l'orifice et le crochet, le bras comprend deux portions rectilignes, avec un léger changement de direction entre les deux portions.

Le document GB2546847 décrit un test urinaire avec une portion de détection colorimétrique (c'est à dire un matériau recouvert d'un réactif chimique pour détecter des composés dans l'urine) attaché temporairement à la cuvette des toilettes par un crochet. L'utilisateur place le test urinaire dans les toilettes juste avant d'uriner, urine directement sur la portion de détection, puis le retire des toilettes pour comparer visuellement la portion de détection colorimétrique avec une référence.

Ces diverses solutions rencontrent toutefois une problématique due aux différentes formes de cuvettes de toilettes qui existent et qui peuvent différer de manière significative en fonction des pays. Par exemple, certaines cuvettes de toilettes présentent un épaulement proche du rebord de la cuvette de toilettes pouvant toucher le bras du crochet et gêner la bonne installation du dispositif d'analyse d'urine. Or, lorsque le dispositif d'analyse d'urine est destiné à rester dans les toilettes pendant plusieurs semaines de suite, il est impératif que l'attache soit optimisée.

### Résumé de l'invention

La présente description propose donc un ensemble d'analyse d'urine permettant une installation aisée et compatible dans différentes formes de cuvettes de toilettes.

A cet effet, la présente description concerne un ensemble pour analyse d'urine comprenant :
- un dispositif d'analyse d'urine configuré pour être placé entièrement dans une cuvette de toilettes et comprenant :
   - un boîtier, le boîtier comprenant :
      * une face avant destinée à recevoir un flux d'urine provenant d'un utilisateur urinant dans la cuvette de toilettes, et
      * une face arrière opposée à la face avant;
   - un élément d'attache disposé sur la face arrière du boîtier,
- un bras de fixation configuré pour positionner le dispositif d'analyse d'urine entièrement dans la cuvette de toilettes, le bras de fixation comprenant une portion d'attache proximale configurée pour coopérer avec l'élément d'attache du dispositif d'analyse d'urine ; dans lequel la portion d'attache proximale forme un espace de réception pour le boîtier, dans lequel l'espace de réception s'étend depuis la face arrière jusqu'au-delà d'un bord de la face arrière.

En effet, un tel ensemble d'analyse d'urine permet de fournir un dispositif d'analyse d'urine offrant une face avant pouvant être vierge pour recevoir un jet d'urine et un élément d'attache disposé sur la face arrière, l'élément d'attache étant ainsi caché et protégé. Le bras de fixation permet de fixer le dispositif d'analyse d'urine dans des cuvettes de toilettes en coopérant avec l'élément d'attache. La forme du bras de fixation formant un espace de réception pour le boîtier depuis l'élément d'attache jusqu'au sommet permet au bras d'épouser la forme du boîtier et ainsi permettre une coopération stable et solide. En outre, cette forme permet de déporter la portion du bras de fixation s'étendant entre le boîtier et le rebord de la cuvette et ainsi éloigner cette portion de la cuvette pour éviter un contact qui risquerait d'entrainer un mauvais positionnement du dispositif d'analyse d'urine. L'ensemble d'analyse d'urine s'adapte ainsi facilement à différents types de formes de cuvettes de toilettes. Ainsi, à l'inverse d'un bras rectiligne qui peut venir au contact de la paroi de cuvette et contraindre le dispositif d'analyse d'urine à être dans une position non adéquate, le bras de fixation tel que décrit ici permet un positionnement adéquat du dispositif d'analyse d'urine.

Dans un mode de réalisation, la face arrière est destinée à faire face à une paroi de la cuvette.

Dans un mode de réalisation, le bras de fixation est amovible.

Dans un mode de réalisation, le bras de fixation est flexible.

Dans un mode de réalisation, le bras de fixation présente une forme aplatie dans une section transverse au plan médian.

Dans un mode de réalisation, le bras de fixation présente une largeur comprise entre 0,5 cm et 20 cm.

Dans un mode de réalisation, la portion d'attache proximale s'étend notamment entre une première extrémité et une deuxième extrémité, dans lequel la première extrémité est au niveau de l'élément d'attache sur la face arrière et la deuxième extrémité est au-delà du bord de la face arrière.

Dans un mode de réalisation, la première extrémité s'étend jusqu'à la face avant du boîtier.

Dans un mode de réalisation, la deuxième extrémité comprend une butée configurée pour bloquer un mouvement du dispositif d'analyse d'urine autrement autorisé par l'élément de fixation.

Dans un mode de réalisation, l'espace de réception est configuré pour recevoir au moins partiellement la partie supérieure de la face arrière du boîtier, jusqu'au sommet du boîtier.

Dans un mode de réalisation, l'espace de réception est de forme complémentaire au boîtier, avec notamment un jeu fonctionnel.

Dans un mode de réalisation, l'élément d'attache est disposé dans la partie supérieure de la face arrière, par exemple entre 15% et 25% en partant du bord de la face arrière.

Dans un mode de réalisation, l'élément d'attache est choisi parmi : un ergot, un aimant, une partie adhésive.

Dans un mode de réalisation, lorsque l'élément d'attache est un ergot/téton, la première portion d'extrémité comprend une cavité (avantageusement traversante) destinée à recevoir l'élément d'attache.

Dans un mode de réalisation, l'ergot s'étend depuis le boîtier selon un corps puis une tête de largeur supérieure au corps.

Dans un mode de réalisation, la cavité présente une première section de largeur transversale maximale supérieure à la largeur de la tête et une deuxième section de largeur transversale maximale inférieure à la largeur de la tête.

Dans un mode de réalisation, le bras de fixation comprend en outre une portion d'attache distale configurée pour coopérer avec la cuvette de toilettes.

Dans un mode de réalisation, la portion d'attache distale comprend un crochet/un aimant/une partie adhésive.

Dans un mode de réalisation, le bras de fixation comprend en outre une portion intermédiaire arrangée entre la portion d'attache proximale et la portion d'attache distale.

Dans un mode de réalisation, la portion d'attache proximale est reliée à la portion intermédiaire au niveau de la deuxième extrémité.

Dans un mode de réalisation, le bras de fixation présente un changement de direction entre la portion intermédiaire et la portion d'attache proximale.

Dans un mode de réalisation, la portion intermédiaire et la portion d'attache proximale forment entre elles au niveau de leur contact un angle (α) inférieure à 90°, notamment inférieure à 60°.

Dans un mode de réalisation, la butée est formée par le changement de direction entre la portion d'attache proximale et la portion intermédiaire.

Dans un mode de réalisation, la portion intermédiaire et la portion d'attache proximale forment entre elles une zone d'inflexion.

Dans un mode de réalisation, la portion intermédiaire et la portion d'attache proximale forment entre elles une arête.

Dans un mode de réalisation, la portion intermédiaire s'étend dans le prolongement du boîtier dans le plan médian du boîtier.

Dans un mode de réalisation, la portion intermédiaire s'étend dans le prolongement de la face avant dans le plan médian du boîtier.

Dans un mode de réalisation, la portion supérieure comprend une surface arrière faisant face à la cuvette et une surface avant opposée à la surface arrière, la surface avant de la portion intermédiaire s'étendant dans le prolongement de la face avant du boîtier.

Dans un mode de réalisation, la portion intermédiaire présente une forme arrondie.

Dans un mode de réalisation, la portion intermédiaire présente une forme convexe.

Dans un mode de réalisation, la forme convexe tend à écarter le dispositif de la cuvette.

Dans un mode de réalisation, le dispositif d'analyse d'urine comprend un orifice de collecte, l'orifice de collecte étant configurée pour collecter le liquide de l'extérieur du boîtier vers l'intérieur du boîtier.

Dans un mode de réalisation, l'orifice de collecte est situé sur la face arrière.

Dans un mode de réalisation, la face arrière comprend au moins un écarteur faisant saillie de la face arrière et configuré pour être en contact avec la cuvette des toilettes.

La présente description concerne également un bras de fixation configuré pour positionner un dispositif d'analyse d'urine entièrement dans une cuvette de toilettes, le dispositif d'analyse comprenant un boîtier comprenant : une face avant destinée à recevoir un flux d'urine provenant d'un utilisateur urinant dans la cuvette de toilettes, et une face arrière opposée à la face avant; et un élément d'attache disposé sur la face arrière du boîtier, le bras de fixation comprenant une portion d'attache proximale configurée pour coopérer avec l'élément d'attache du dispositif d'analyse d'urine ; dans lequel la portion d'attache proximale forme un espace de réception pour le boîtier, dans lequel l'espace de réception s'étend depuis la face arrière jusqu'au-delà d'un bord de la face arrière.

### Présentation des figures

D'autres caractéristiques, détails et avantages apparaîtront à la lecture de la description détaillée ci-après, et à l'analyse des dessins annexés, sur lesquels :
- [FIG. 1] : la figure 1 présente une représentation schématique et simplifiée d'un dispositif d'analyse d'urine installé dans une cuvette de toilettes,
- [FIG. 2] : la figure 2 présente une vue éclatée du dispositif d'analyse d'urine, dans laquelle la station et la cartouche sont visibles,
- [FIG. 3] : la figure 3 présente une vue détaillée d'une cartouche selon un mode de réalisation,
- [FIG. 4] : la figure 4 présente une vue en coupe d'une cartouche et d'une station selon un mode de réalisation, à l'emplacement d'un analyseur optique de la station,
- [FIG. 5] : la figure 5 présente une vue latérale d'un ensemble d'analyse selon un mode de réalisation de la description avec un bras de fixation dans une configuration de repos,
- [FIG. 6] : la figure 6 présente une vue latérale d'un ensemble d'analyse selon un mode de réalisation de la description avec un bras de fixation dans une configuration déployée,
- [FIG. 7] : la figure 7 présente une vue en perspective (a) et une vue latérale (b) d'un bras de fixation de l'ensemble des figures 5 et 6,
- [FIG. 8] : la figure 8 présente une vue latérale partielle (a) d'un dispositif d'analyse d'urine et une vue de face (b) du bras de fixation de l'ensemble des figures 5 et 6,
- [FIG. 9] : la figure 9 présente une vue latérale d'un ensemble d'analyse selon un autre mode de réalisation de la description.

### Description détaillée

La présente description présente différents exemples de dispositif d'analyse d'urine comprenant une station et une cartouche telle que divulguée dans les documents WO2021/175909 et WO2021/175944, ci-après dénommés WO'909 et WO'944. Des variantes des stations sont présentées dans les documents WO2023036805, WO2023036806, WO2023036808, WO2023036809, ci-après dénommés WO'80X.

Les paragraphes suivants expliquent le principe général d'un dispositif d'analyse d'urine, mais tous les détails des documents WO'909 et WO'933 (ainsi que de tous les documents PCT susmentionnés) sont applicables.

### Orientation

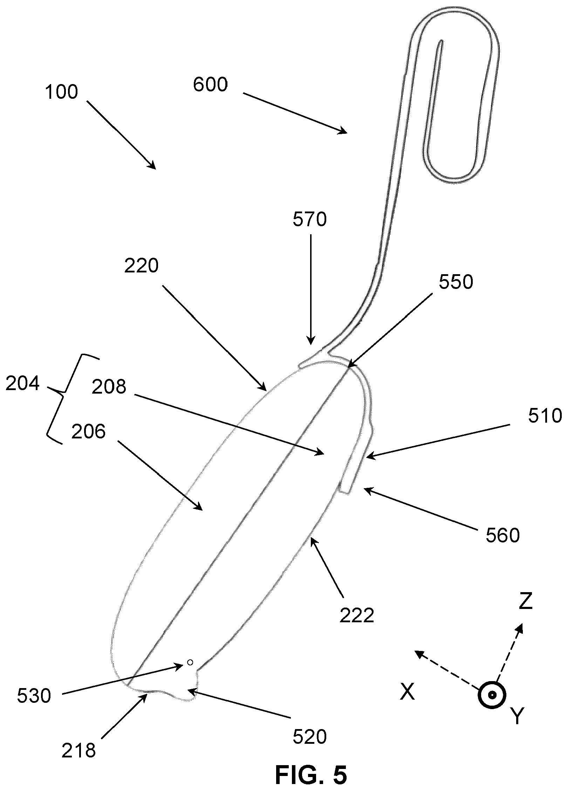

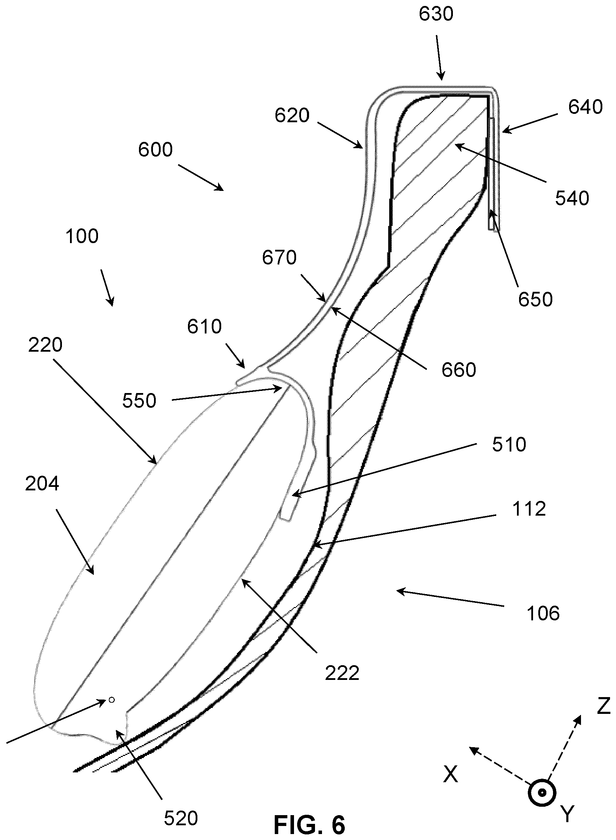

Dans la suite de l'exposé, les notions de "haut" et "bas", "supérieur" et "inférieur", etc. sont définies par rapport à une direction Z, telle que définie sur les figures 5 et 6. Le haut selon la direction Z est défini dans une position d'utilisation normale du dispositif d'analyse d'urine fixé dans la cuvette des toilettes.

### Forme générale du boîtier

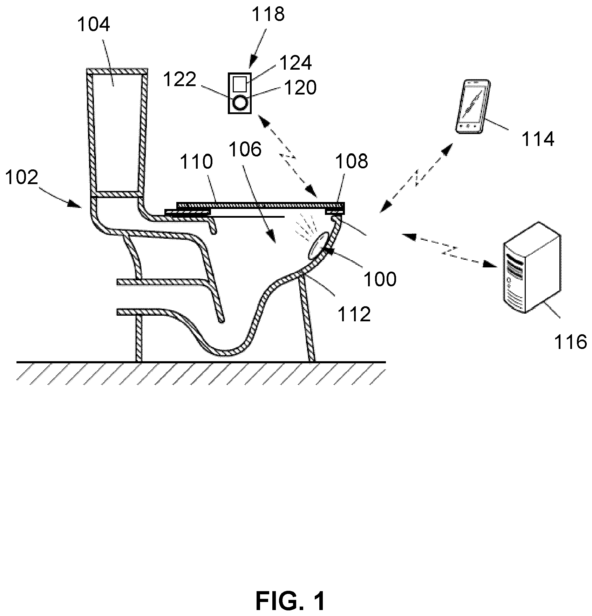

La figure 1 illustre schématiquement un dispositif d'analyse 100 (appelé "dispositif 100") pour l'analyse d'urine installé dans les toilettes 102. Les toilettes 102 comprennent généralement un réservoir d'eau 104, une cuvette 106, un siège 108 et un couvercle de siège 110. Le dispositif d'analyse 100 est configuré pour être placé entièrement dans la cuvette des toilettes. Par "dans la cuvette", on entend "placé dans le volume intérieur défini par la cuvette". Le dispositif d'analyse 100 est disposé de manière amovible dans les toilettes 102. Par exemple, le dispositif d'analyse 100 peut être facilement retiré des toilettes pour remplacer une cartouche, puis replacé dans les toilettes 102. Le dispositif d'analyse 100 est placé sur une paroi interne 112 de la cuvette 106 des toilettes. Le dispositif d'analyse 100 est placé de telle sorte qu'il se trouve généralement sous le jet d'urine d'un utilisateur, de sorte que lorsqu'un utilisateur urine (généralement en position assise), l'urine entre en contact avec le dispositif d'analyse 100. Le dispositif d'analyse 100 peut communiquer à distance avec une entité distante, telle que le smartphone 114 ou un serveur 116.

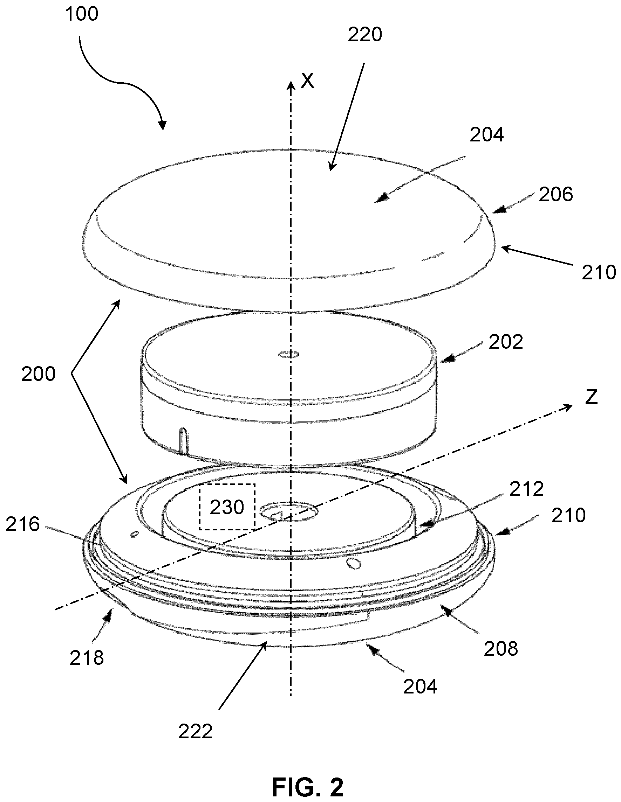

Comme l'illustre plus en détail la figure 2, le dispositif d'analyse 100 peut comprendre une station 200 et une cartouche 202, montée de manière amovible sur la station 200. La station 200 peut comprendre un boîtier 204 qui peut comporter deux coques, notamment une coque avant 206 et une coque arrière 208. La coque avant 206 et la coque arrière 208 peuvent coopérer l'une avec l'autre par l'intermédiaire d'un mécanisme de fixation 216, dans un plan normal à l'axe X. La coque avant 206 et la coque arrière 208 peuvent être assemblées de manière réversible, par exemple par vissage ou par clipsage. Dans une variante, la coque avant 206 et la coque arrière 208 peuvent être assemblées de manière permanente, par exemple par collage, clipsage, magnétisation, ou soudage par ultrasons. Bien entendu, d'autres moyens de fixation peuvent être utilisés pour assembler les deux coques.

En particulier, comme illustré sur la figure 2, la coque avant 206 et la coque arrière 208 sont vissées l'une à l'autre. Ensuite, une partie interne de la coque avant 206 comprend un filetage. Le filetage de la coque avant 206 est destiné à coopérer avec un filetage complémentaire de la coque arrière 208. Cela permet de démonter facilement le boîtier 204 pour accéder à l'assemblage de test à l'intérieur du boîtier.

Un joint peut être présent entre la coque avant 206 et la coque arrière 208. Ainsi, le boîtier 204 est étanche. Seuls les orifices de collecte et de drainage relient l'extérieur et l'intérieur de la boîtier 204, comme décrit plus en détail ci-dessous.

Comme on peut le voir sur les figures, le boîtier 204 peut avoir une forme globale extérieure de galet circulaire. En d'autres termes, le boîtier 204 présente une forme sphéroïde. L'axe X est la ligne centrale du boîtier. Avantageusement, la coque avant 206 peut être sensiblement symétrique en rotation, ce qui donne un aspect aérodynamique au dispositif une fois installé. Le boîtier 204 sert de collecteur d'urine.

Le boîtier 204 comprend une face avant 220 pour recevoir un flux d'urine directement d'un utilisateur urinant sur les toilettes et une face arrière 222 opposée à la face avant 220. Comme illustré à la figure 2, la face avant 220 peut être disposée sur la coque avant 206 et la face arrière 222 peut être disposée sur la coque arrière 208. La face avant 220 est orientée vers l'intérieur de la cuvette 106. La face avant 220 est donc destinée à recevoir l'urine lorsque l'utilisateur urine en s'asseyant sur les toilettes 102. Comme le montrent les figures 5 et 6, la face arrière 222 fait face à la paroi intérieure 112 de la cuvette 106. Par la suite de la description, par un objet faisant face à la paroi de la cuvette, il est entendu un objet faisant face à la paroi de la cuvette la plus proche de l'objet en question, et non la paroi de la cuvette en vis-à-vis de l'autre côté du volume interne de la cuvette.

La face avant 220 et la face arrière 222 peuvent comporter chacune un bord incurvé 210. Les bords incurvés 210 respectifs de la face avant et de la face arrière se rejoignent au niveau d'une zone de jonction équatoriale. Ainsi, la surface extérieure du boîtier 204, constituée de la face avant 220 et de la face arrière 222, est définie par des lignes courbes et forme un objet généralement convexe.

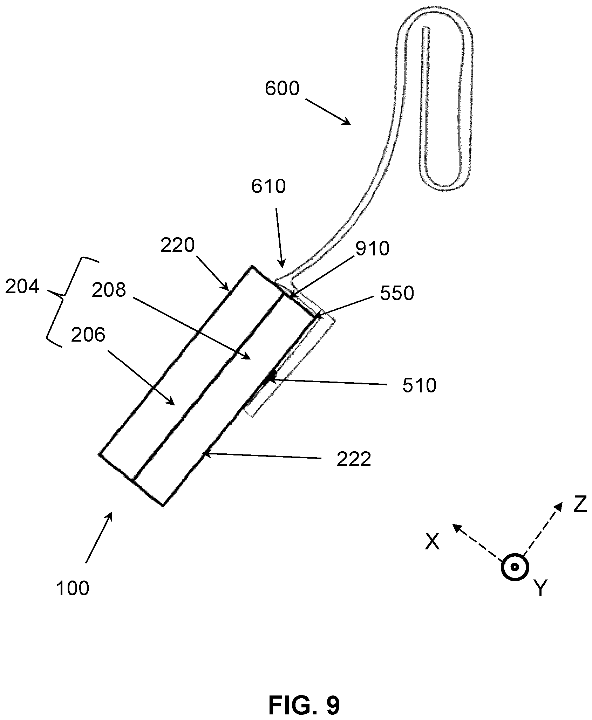

En référence aux figures 5 et 6, le face arrière 222 présente un bord 550. Le bord 550 est l'endroit où la face arrière s'arrête et où commence soit une face de transition, soit la face avant. Le bord 550 passe typiquement à proximité d'un point le plus haut du boitier 204 selon l'axe Z dans une position d'utilisation normale du boitier 204 lorsqu'il est placé dans la cuvette 106 des toilettes. Dans le mode de réalisation en galet à deux coques, le bord 550 est à la jonction entre la face avant 220 et de la face arrière 222. Dans un mode de réalisation où le boitier 204 est de forme parallélépipédique comme illustré sur la figure 9, le bord 550 est situé à la jonction entre la face arrière 222 et une face supérieure 910, qui relie la face avant 220 et la face arrière 222.

La surface extérieure de la face avant 220 peut être lisse. En d'autres termes, la face avant 220 est dépourvue de crêtes ou de rainures. Ainsi, le flux d'urine entrant en contact avec la face avant 220 s'accroche et s'étale sur la face avant 220. La face avant 220 peut être sensiblement symétrique en rotation autour de l'axe X.

La surface extérieure du boîtier 204 peut également être blanche ou de couleur claire. La couleur de la surface extérieure peut être similaire à celle des toilettes, ce qui accroît la discrétion du dispositif.

Le boîtier 204 peut avoir un diamètre, mesuré dans la direction orthogonale à l'axe X, compris entre 50 mm et 150 mm. Le boîtier 204 peut avoir une épaisseur, mesurée dans la direction de l'axe X, comprise entre 15 mm et 50 mm. Ainsi, le boîtier 204 est suffisamment compact pour être entièrement logé dans la cuvette des toilettes. Le dispositif d'analyse d'urine 100 est discret. De plus, le boîtier 204 est suffisamment grand pour entrer systématiquement en contact avec l'urine reçue dans la cuvette. L'utilisateur peut alors uriner dans les toilettes sans se soucier du dispositif d'analyse d'urine, ou alternativement viser sommairement.

Selon un autre aspect, dans un mode de réalisation, le boîtier 204 présente un facteur de forme général tel que le rapport entre l'épaisseur et le diamètre est compris entre 0,2 et 0,5, et même de préférence entre 0,3 et 0,4. De telles proportions rappellent un galet naturel et donnent au dispositif un aspect apaisant.

De préférence, le boîtier 204 est constitué d'un matériau hydrophile. Par exemple, le matériau du boîtier 204 peut être : une céramique, un polyamide (PA), un silicone ou un polymère hydrophile. La surface extérieure du boîtier 204 peut également être traitée avec un traitement de surface hydrophile, par exemple acuWet^{®} d'Aculon, un polymère hydrophile, ou Pebax^{®} d'Arkema.

### Ensemble de test

Un ensemble de test 230 est disposé à l'intérieur du boîtier 204 et configuré pour effectuer une analyse sur l'urine collectée par l'orifice de collecte. La station 200 comprend un compartiment annulaire 212, situé à l'intérieur du boîtier 204, disposé autour d'un axe de rotation X. Le compartiment annulaire 212 est configuré pour recevoir au moins partiellement la cartouche 202 montée de manière rotative autour de l'axe de rotation X (une fois en position dans le compartiment annulaire 212). La cartouche 202 comprend une pluralité de supports de test qui comprennent chacun au moins un réactif urinaire, par exemple un réactif sec, la pluralité de supports de test étant disposée le long d'un cercle ou d'un arc de cercle autour de l'axe de rotation X. Dans un mode de réalisation, les supports de test sont des bandelettes de test. Les supports de test peuvent être enfermés, par exemple individuellement, dans une chambre.

Le compartiment annulaire 212 s'étend typiquement sur 360° et forme une gorge configurée pour recevoir au moins partiellement la cartouche 202.

La station 200 comprend un orifice de collecte 218, située par exemple sur la coque arrière 208. Comme cela sera expliqué plus en détail ci-dessous, l'orifice de collecte 218 est configurée pour collecter l'urine s'écoulant sur la surface du boîtier 204. La station 200 comprend également un orifice de drainage 530, visible sur la figure 5, configurée pour drainer le liquide hors du dispositif 100.

L'ensemble de test 230 peut comprendre une pompe, un injecteur et un analyseur. La pompe aspire l'urine de l'orifice de collecte 218, puis l'injecteur injecte l'urine sur un ou plusieurs supports de test de la cartouche et l'analyseur obtient certaines valeurs de propriétés (par exemple, des propriétés physiques/chimiques, telles que la couleur) des supports de test après qu'ils sont entrés en contact avec l'urine. Dans un cas, l'analyseur est un analyseur optique configuré pour analyser les propriétés optiques du support de test. L'injecteur et la cartouche peuvent se déplacer l'un par rapport à l'autre afin que l'injecteur puisse ouvrir (par exemple, percer) la chambre, par exemple à l'aide d'une aiguille ou d'un dispositif semblable à une aiguille.

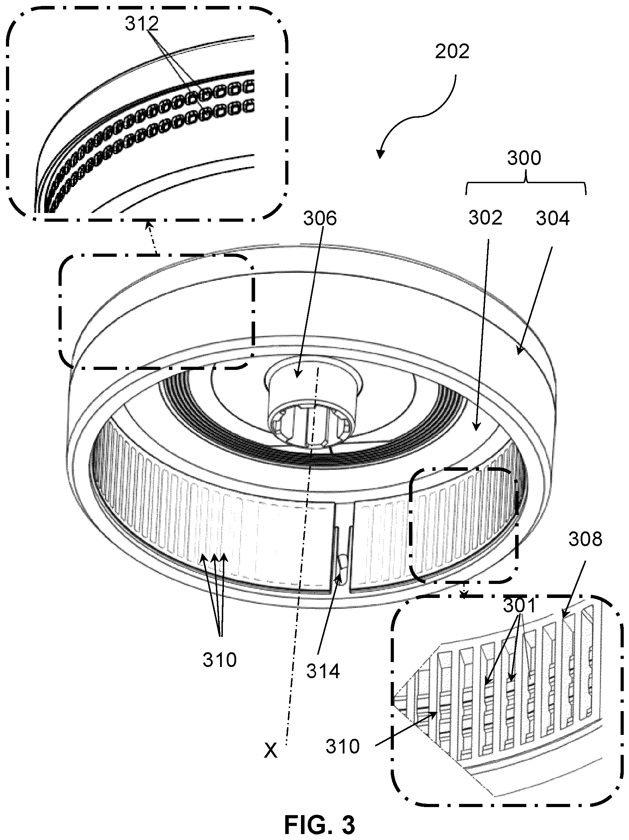

La figure 3 montre une vue éclatée de la cartouche 202. La cartouche 202 comprend au moins un support de test 301, notamment plusieurs supports de test 301 configurés pour recevoir l'urine de l'injecteur. Chaque support de test 301 contient un réactif urinaire qui réagit de manière spécifique au contact de l'urine. La cartouche 202 comprend un support rotatif 300, configuré pour être entraîné en rotation par la station 200. Dans le cadre d'une utilisation normale de la cartouche 202 et du dispositif 100, les supports de test 301 restent fixés au support rotatif et ne se déplacent pas par rapport à ce dernier.

Dans une réalisation, le support rotatif 300 a une forme de cylindre circulaire droit d'au moins 80% d'une forme de cylindre creux s'étendant annulairement autour d'un axe qui est, lorsque la cartouche 202 est montée dans la station 200, l'axe de rotation X. Chaque support de test 301 peut être une bandelette de test. Le support rotatif 300 peut comprendre une partie annulaire 302 et une partie cylindrique 304, qui s'étend à partir d'une extrémité radiale extérieure de la partie annulaire 302. La partie cylindrique 304, lorsqu'elle est utilisée, est logée à l'intérieur du compartiment annulaire 212. Les supports de test 301 sont positionnés le long de la portion cylindrique 304, de manière à pouvoir défiler sélectivement et/ou successivement devant l'injecteur et l'analyseur. Par exemple, les supports de test 301 font partie d'un support 308, qui comprend plusieurs chambres 310, séparées les unes des autres le long d'un périmètre autour de l'axe X. Au moins une bandelette de test est reçue dans une chambre 310.

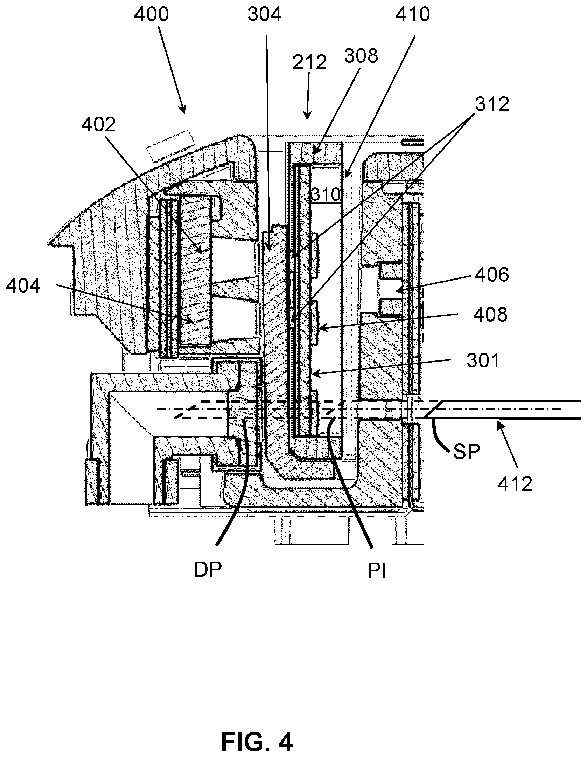

Les chambres 310 sont disposées les unes à côté des autres en forme de cylindre circulaire droit d'au moins 80 % du cercle. Pour permettre à la lumière de passer, le support 308 comprend au moins une ouverture 312 par chambre 310 (représentée dans le zoom supérieur gauche où le support rotatif est représenté comme transparent). Les chambres 310 sont toutes à égale distance de l'axe de rotation X, de sorte que l'injecteur peut injecter sélectivement l'urine une fois que la chambre souhaitée est positionnée à l'endroit voulu face à l'injecteur. L'injecteur peut se déplacer vers la chambre 310 et percer un couvercle fermant la chambre 310 (visible sur la figure 4). Un orifice de vidange 314 est prévue dans le support rotatif 300 pour permettre l'évacuation de l'urine de l'injecteur vers l'extérieur du dispositif 100, via l'orifice de drainage 530 disposée sur le boîtier 204.

La partie annulaire 302 du support rotatif 300 reste à l'extérieur du compartiment annulaire 212 pour renforcer la partie cylindrique et/ou entraîner en rotation la cartouche 202. À cette fin, la partie annulaire 302 peut comprendre un accouplement mécanique 306, qui coopère avec un arbre de la station 200.

Les dimensions relatives à la cartouche 202 sont divulguées dans les documents WO'909, WO'933 et WO'80X. La dimension maximale du dispositif 100 transversalement à l'axe de rotation X est inférieure à 15 cm, voire inférieure à 10 cm. La dimension maximale du dispositif le long de l'axe de rotation X est inférieure à 5 cm.

La figure 4 montre plus en détail l'interaction entre la cartouche 202 et la station 200 lorsque ou après l'activation de l'injecteur. L'analyseur 400 comprend au moins une source lumineuse 402, 404 (par exemple, deux sources lumineuses) et au moins un capteur optique 406. La lumière va de la source lumineuse 402, 404 au capteur optique 406 en traversant la cartouche 202 et en particulier la partie cylindrique 304, l'ouverture 312 du support 308, le support de test 301 et donc le réactif 408.

Dans un mode de réalisation, l'analyseur 400 est configuré pour mesurer l'absorbance d'une partie des supports de test 301.

L'absorbance est détectée par la source lumineuse (par exemple une LED) qui peut faire passer de la lumière à travers la bande, et le capteur optique qui reçoit le spectre avec une dizaine de longueurs d'onde.

Dans une variante, le capteur de lumière est une caméra capable de détecter un changement de couleur, notamment un changement d'intensité de couleur, d'une partie des supports de test 301.

La caméra peut détecter une couleur en valeurs RVB par exemple.

L'injecteur comprend une extrémité d'injection 412 (par exemple une aiguille), qui peut être déplacée entre plusieurs positions, représentées par des lignes en tirets sur la figure 4. Dans une position d'attente SP, l'extrémité d'injection 412 est à l'extérieur de la cartouche 202 (dans une position la plus intérieure), de sorte que la cartouche 202 peut tourner librement dans le compartiment annulaire 212 ; dans une position d'injection IP, l'extrémité d'injection 412 a percé le couvercle 410 pour accéder à l'intérieur de la chambre 310 et peut injecter un peu d'urine sur le support de test 301 ; dans la position de vidange DP, l'extrémité d'injection 412 est capable d'évacuer l'urine par l'orifice de vidange 314 du support rotatif 300.

En position SP, l'injecteur est situé radialement à l'intérieur de la chambre annulaire. Ceci permet de maximiser le rayon du compartiment annulaire tout en minimisant la taille de la station 200.

Dans une variante de l'ensemble de test 230 divulgué ci-dessus, l'homme de métier comprendra que chaque ensemble de test configuré pour analyser l'échantillon d'urine collecté par l'orifice de collecte 218 peut être disposé à l'intérieur du boîtier 204.

### Orifice de collecte

L'orifice de collecte 218 est configuré pour recevoir l'urine qui s'écoule par gravité sur la surface extérieure du boîtier 204. L'urine est collectée directement sur la face avant 220 et la face arrière 222 du boîtier 204.

L'orifice de collecte 218 est une ouverture configurée pour collecter le liquide, de sorte que le liquide puisse pénétrer dans le dispositif d'analyse d'urine. L'orifice de collecte 218 est généralement circulaire, avec un diamètre de préférence compris entre 0,3 mm et 2 mm. Le diamètre de l'orifice de collecte peut être choisi pour maximiser le volume d'urine collecté sur la surface extérieure du boîtier 204.

Comme on peut le voir sur les figures, l'orifice de collecte 218 est situé sur la face arrière 222. Ainsi, l'orifice de collecte 218 fait face à la paroi interne 112 des toilettes lorsque le dispositif d'analyse d'urine 100 est positionné dans les toilettes. Cette position permet à l'orifice de collecte 218 d'être cachée à la vue de l'utilisateur par la face avant 220 du boîtier. La face avant 220 visible par l'utilisateur ressemble à un simple galet uniforme, comme déjà mentionné, sans points singuliers ni trous. Il convient également de noter que cette position empêche l'introduction de contaminants ou d'éléments qui pourraient obstruer l'ensemble de test 230.

L'orifice de collecte 218 est situé sur une partie inférieure de la face arrière 222. Par "sur une partie inférieure de la face arrière", on entend "sur le dernier quart de la face selon la direction Z à partir de l'extrémité inférieure du boîtier 204". L'extrémité inférieure fait face au fond de la cuvette 106 lorsque le boîtier 204 est positionné dans la cuvette. L'extrémité inférieure se situe à l'opposé du sommet 550. Cette position correspond à une position normale d'utilisation. Cette position permet à l'urine d'être collectée par gravité sur la majeure partie de la surface extérieure du boîtier 204.

En particulier, la distance séparant l'orifice de collecte 218 d'un bord inférieur du boîtier 204 est inférieure à 40 mm, de préférence inférieure à 20 mm. Comme illustré, selon un mode de réalisation particulier, l'orifice de collecte 218 est disposée à quelques millimètres au-dessus du bord inférieur du boîtier 204. En variante, l'orifice de collecte 218 peut se trouver sur le bord inférieur (le bord inférieur étant défini lorsque le dispositif 100 est placé pour être utilisé dans les toilettes).

L'orifice de collecte 218 peut être couvert par un filtre à mailles. Le filtre à mailles est par exemple de forme oblongue et recouvre l'orifice de collecte 218. L'ouverture moyenne des mailles du filtre est, par exemple, de 20 microns. Le filtre à maille empêche l'introduction de contaminants ou d'éléments susceptibles d'obstruer l'ensemble de test 230 et filtre l'urine reçue dans l'orifice de collecte 218. La maille du filtre peut être en métal.

### Ecarteur

En référence aux figures 5 et 6 notamment, dans un mode de réalisation, le dispositif 100 peut comprendre au moins un écarteur 520 faisant saillie de la face arrière 222 et configuré pour être en contact avec la cuvette 106, en particulier en contact avec la paroi interne 112 de la cuvette 106. L'écarteur 520 est disposé sur la face arrière 222. Dans un mode de réalisation, l'écarteur 520 est disposé sur la face arrière 222 de sorte que l'orifice de collecte 218 se trouve à une certaine distance de la cuvette 106 lors d'une utilisation normale du dispositif. Cela évite que la paroi interne 112 de la cuvette 106 n'obstrue l'entrée de l'orifice de collecte 218 et/ou ne constitue un goulot d'étranglement pour l'écoulement d'un mélange d'urine, d'eau et d'impuretés provenant de la cuvette (qui pourrait être collecté par l'orifice de collecte dans d'autres circonstances).

Grâce à l'au moins un écarteur 520, l'urine s'écoule sur le boîtier 204 (en particulier sur la face arrière 222) vers l'orifice de collecte 218 sans être gênée par la cuvette 106 comme cela pourrait se produire avec un dispositif tel que décrit dans les documents WO'909 et WO'944.

Dans un mode de réalisation, les écarteurs 520 sont situés sur la partie inférieure du dispositif d'analyse d'urine 100, et plus particulièrement sur la partie inférieure de la face arrière 222, à côté de l'orifice de collecte 218.

L'écarteur 520 comprend typiquement une arête. Lors de l'utilisation, une partie de l'arête est en contact avec la cuvette des toilettes.

En particulier, le dispositif 100 peut comprendre deux écarteurs 520, avantageusement disposés de chaque côté de l'orifice de collecte 218, le long de la direction transversale Y. Les deux écarteurs 520 peuvent être disposés de chaque côté de l'orifice de collecte 218, le long de l'orifice de collecte 218. Les deux écarteurs 520 peuvent être symétriques par exemple le long d'un plan vertical XZ, dans une position d'utilisation normale du dispositif 100 ou par exemple autour de l'orifice de collecte 218.

L'écarteur 520 a été décrit en détail dans la demande de brevet EP23192265.

### FIXATION

Le boîtier 204 est destiné à être placé sur la paroi interne 112 de la cuvette 106. Le boîtier 204 est positionné et maintenu en position dans la cuvette par un bras de fixation 600. Le bras de fixation 600 est configuré pour fixer le dispositif d'analyse d'urine 100 dans la cuvette 106 des toilettes. Le bras de fixation 600 comprend plusieurs sections qui ont des fonctions différentes et qui seront décrites en détail par la suite. Toutefois, le bras de fixation 600 est typiquement réalisé en un seul matériau, par exemple du plastique, par moulage.

Le dispositif d'analyse 100 comprend un élément d'attache 510 disposé sur la face arrière 222 du boîtier. L'élément d'attache 510 est configuré pour coopérer avec le bras de fixation 600. Avec un élément d'attache 510 sur la face arrière 222, la face avant 220 peut rester vierge et ainsi éviter que le jet d'urine soit accidentellement réfléchi en direction de l'ouverture de la cuvette des toilettes. En effet, la distance entre l'urètre et le dispositif d'analyse d'urine, ainsi que la pression du jet d'urine, font que tout aspérité sur la face avant 220 qui reçoit le jet peut provoquer un éclaboussement.

L'élément d'attache 510 est par exemple disposé au niveau d'un plan médian XZ du boîtier 204. En d'autres termes, l'élément d'attache 510 est disposé au niveau d'un plan de symétrie vertical du boitier 204.

Comme on peut le voir sur les figures, l'élément d'attache 510 peut être disposé sur la partie supérieure du boîtier 204. Par "sur la partie supérieure du boîtier", on entend "sur le premier quart le long de la direction Z à partir du sommet du boîtier 204". En particulier, l'élément d'attache 510 est disposé entre 15% et 25% de la face arrière 222 en partant du sommet 550 du boîtier 204.

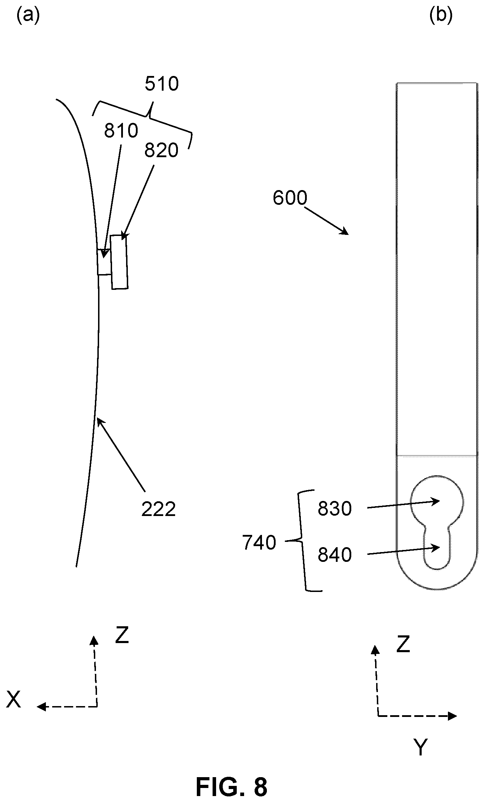

Comme visible sur la figure 8, l'élément d'attache 510 peut être un ergot. L'ergot fait saillie hors de la face arrière 222 du boitier 204. Selon un mode de réalisation, l'ergot s'étend depuis la face arrière 222 par un corps 810 puis par une tête 820 d'étendue transversale supérieure au corps 810.

En variante non représentée, l'élément d'attache 510 est un aimant propre à coopérer avec un autre aimant placé sur le bras de fixation 600.

En variante non représentée, l'élément d'attache 510 comprend une partie adhésive propre à coopérer avec le bras de fixation 600.

### Bras de fixation

Selon un mode de réalisation, le bras de fixation 600 est amovible du dispositif d'analyse d'urine. Autrement dit, l'utilisateur peut séparer le bras de fixation 600 de l'élément d'attache 510. Le bras de fixation 600 peut être ainsi aisément remplacé et une autre forme de bras de fixation peut être par exemple utilisée.

Le bras de fixation 600 peut être flexible. Autrement dit, le bras de fixation 600 peut être déformé par l'utilisateur ou par la gravité du dispositif 100 placé dans les toilettes. Ceci permet ainsi une meilleure adaptation du bras de fixation aux différentes formes de toilettes et assure que le boitier 204 touche la paroi interne 112 des toilettes. En particulier, le bras de fixation 600 est fait en un matériau plastique, par exemple du nylon ou un matériau métallique, par exemple de l'acier.

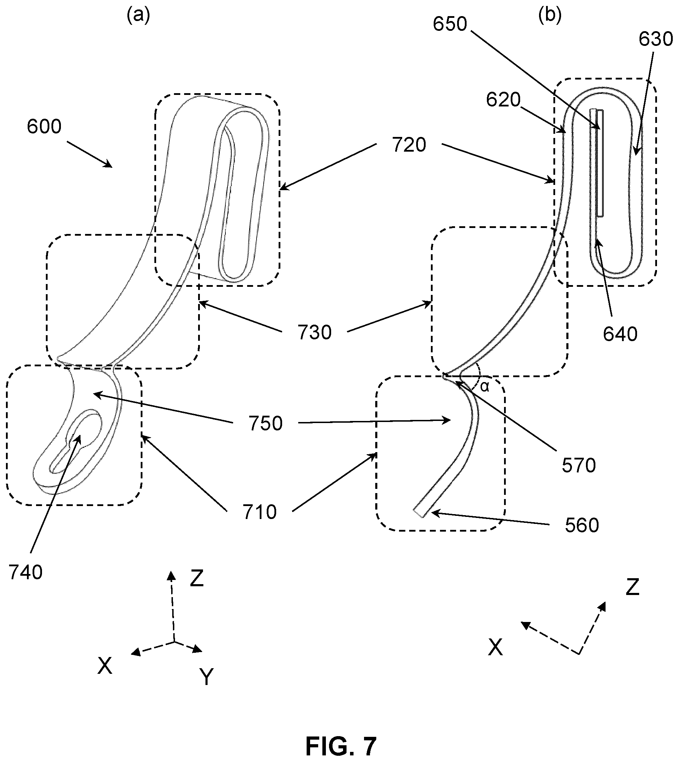

Comme visible sur la figure 7, le bras de fixation 600 peut présenter une forme aplatie dans une section transverse au plan médian XZ. Ceci permet une flexibilité du bras de fixation 600 dans le plan médian XZ tout en limitant la flexibilité selon la direction transversale Y.

Le bras de fixation 600 peut s'étendre selon une hauteur selon l'axe Z comprise entre 5 cm et 15 cm. Le bras de fixation 600 présente par exemple une largeur selon l'axe Y comprise entre 1 cm et 2 cm. Le bras de fixation 600 présente par exemple une épaisseur comprise entre 1 mm et 2 mm.

### Portion d'attache proximale

En référence aux figures 6 et 7, le bras de fixation 600 comprend une portion d'attache proximale 710 configurée pour coopérer avec l'élément d'attache 510 du dispositif d'analyse d'urine 100. Par la suite, les termes "proximal" et "distal" sont définis selon la proximité au dispositif d'analyse 100.

Lorsque l'élément d'attache 510 est un ergot, la portion d'attache proximale 710 comprend une cavité 740 destinée à recevoir l'élément d'attache 510. La cavité 740 peut être traversante.

En référence à la figure 8, la cavité 740 peut présenter une première section 830 de largeur transversale maximale supérieure à la largeur de la tête 820 de l'ergot et une deuxième section 840 de largeur transversale maximale inférieure à la largeur de la tête 820 de l'ergot. La deuxième section 840 est également de largeur transversale supérieur au corps 810 de l'ergot. Ainsi, lorsque l'utilisateur veut assembler le bras de fixation 600 avec le dispositif 100, il/elle introduit l'ergot dans la première section 830 puis fait glisser l'ergot dans la deuxième section 840. La tête 820 de l'ergot étant plus large que la deuxième section 840, le bras de fixation 600 est maintenu solidaire du dispositif 100. A l'inverse, lorsque l'utilisateur veut désolidariser le bras de fixation 600 du dispositif 100, il/elle glisse l'ergot de la deuxième section 840 vers la première section 830 afin de permettre le passage de la tête 820 de l'ergot à travers la cavité 740.

La portion d'attache proximale 710 comprend une première extrémité 560 au niveau de l'élément d'attache 510 et une deuxième extrémité 570 qui est au-delà du bord 550 de la face arrière 222 (lorsque le bras de fixation 600 est associé au boitier 204). Dans un mode de réalisation, la deuxième extrémité 570 se trouve au niveau de la face avant 220 du boîtier 204. Comme illustré sur les figures, la portion d'attache proximale 710 s'étend essentiellement entre ces deux extrémités 560, 570.

### Espace de réception

Comme visible sur les figures 5 à 9, la portion d'attache proximale 710 forme un espace de réception 750 pour le boîtier 204. Lorsque le bras de fixation 600 coopère avec le boitier 204, l'espace de réception 750 s'étend depuis l'élément d'attache 510 jusqu'à au moins un bord 550 de la face arrière 222 du boîtier 204. L'espace de réception 750 s'étend ainsi depuis la face arrière 222 jusqu'au moins le bord 550. Dans un mode de réalisation, l'espace de réception 750 s'étend jusqu'à la face avant 220 du boitier 204, comme illustré sur la figure 6. L'espace de réception 750 est ainsi délimité par la première extrémité 560 et la deuxième extrémité 570.

L'espace de réception 750 est configuré pour recevoir au moins partiellement le boîtier 204, dont notamment la partie supérieure de la face arrière 222, jusqu'au bord 550. Dans un mode de réalisation, l'espace de réception 750 est de forme complémentaire au boîtier, notamment à la partie supérieure de la face arrière 222 du boitier 204, avec par exemple un jeu fonctionnel lorsque le dispositif 100 est installé dans la cuvette 106 des toilettes.

Dans un mode de réalisation, comme illustré sur les figures 5 à 8, l'espace de réception 750 est de forme arrondie, notamment de forme complémentaire avec la forme en galet du boitier 204. En variante, comme illustré sur la figure 9, l'espace de réception 750 présente une forme anguleuse, notamment de forme complémentaire avec la forme parallélépipédique du boitier 204.

Dans un mode de réalisation, la portion d'attache proximale 710, et plus particulièrement la deuxième extrémité 570, comprend une butée 610. La butée 610 définit une extrémité de l'espace de réception 750 opposée à l'extrémité de l'espace de réception coopérant avec l'élément d'attache 510. La butée 610 est configurée pour bloquer un mouvement du dispositif d'analyse d'urine 100 autorisé par le bras de fixation 600. Ce blocage se fait notamment par contact avec la face avant ou la face de transition.

### Portion d'attache distale

Le bras de fixation 600 comprend en outre une portion d'attache distale 720. La portion d'attache distale 720 est opposée à la portion d'attache proximale 710. Comme visible sur la figure 6, la portion d'attache distale 720 est configurée pour coopérer avec la cuvette 106 de toilettes.

Dans un mode de réalisation illustré sur les figures, la portion d'attache distale 720 comprend un crochet. En particulier, la portion d'attache distale 720 est mobile d'une configuration de repos, comme illustré sur les figures 5 et 7, dans laquelle la portion d'attache distale 720 est repliée sur elle-même à une configuration déployée, comme illustré sur la figure 6, dans laquelle la portion d'attache distale 720 est déployée. Dans la configuration déployée, la portion d'attache distale 720 est configurée pour être accrochée au rebord 540 de la cuvette 106 de toilettes. La portion d'attache distale 720 comprend trois parties allongées successives 620, 630, 640. Comme visible sur la figure 7, dans la configuration de repos, la troisième partie 640 est repliée entre la première partie 620 et la deuxième partie 630. Les trois parties 620, 630, 640 sont alors sensiblement parallèles entre elles. Comme visible sur la figure 6, dans la position déployée, la deuxième partie 630 et la troisième partie 640 sont à l'écart de la première partie 620, chaque paire de parties successives formant sensiblement un angle droit entre elles.

La troisième partie 640 peut comprend un bloc antidérapant 650, par exemple en gomme, permettant une meilleure adhérence du bras de fixation 600 avec la cuvette 106.

En variante non représentée, le bras de fixation 600 comprend un aimant propre à coopérer avec un autre aimant placé notamment sur l'élément d'attache 510.

En variante non représentée, le bras de fixation 600 comprend une partie adhésive propre à coopérer avec l'élément d'attache 510.

### Portion intermédiaire

Le bras de fixation 600 comprend en outre une portion intermédiaire 730 arrangée entre la portion d'attache proximale 710 et portion d'attache distale 720.

La portion d'attache proximale 710 est reliée à la portion intermédiaire 730 au niveau de la deuxième extrémité 570. Dans cette configuration, le bras de fixation 600 ne comporte pas de section en embranchement, mais uniquement à la suite des autres.

La portion intermédiaire 730 et la portion d'attache proximale 710 forment entre elles une zone d'inflexion. Autrement dit, le bras de fixation 600 présente un changement de direction entre la portion intermédiaire 730 et la portion d'attache proximale 710. En d'autres termes, la portion intermédiaire 730 et la portion d'attache proximale 710 forment entre elles une arête. La butée 610 est notamment formée par ce changement de direction. En particulier, en référence à la figure 7, la portion intermédiaire 730 et la portion d'attache proximale 710 forment entre elles au niveau de leur contact un angle α inférieur à 90°, notamment inférieure à 60°.

La portion intermédiaire 730 s'étend dans le prolongement du boitier 204 dans le plan médian XZ du boîtier 204. En particulier, la portion intermédiaire 730 comprend une surface arrière 660 faisant face à la paroi interne 112 de la cuvette 106 et une surface avant 670 opposée à la surface arrière 660. La surface avant 670 s'étend dans le prolongement de la face avant 220 du boîtier, notamment pour favoriser le passage de l'urine sur le boitier. Autrement dit, le plan tangent au boitier au niveau de la butée 610 est sensiblement parallèle au plan tangent à la surface avant 670 de la portion intermédiaire 730 au niveau de la butée 610.

Comme visible sur les figures, la portion intermédiaire 730 peut présenter une forme arrondie. En particulier, la portion intermédiaire 730 peut présenter une forme convexe, autrement dit courbée vers la cuvette. La forme convexe de la portion intermédiaire 730 tend à écarter le dispositif 100 de la cuvette 106, comme cela est visible sur la figure 6.

## Revendications

1. Ensemble pour analyse d'urine comprenant :
- un dispositif d'analyse d'urine (100) configuré pour être placé entièrement dans une cuvette (106) de toilettes et comprenant :
∘ un boîtier (204), le boîtier (204) comprenant :
▪ une face avant (220) destinée à recevoir un flux d'urine provenant d'un utilisateur urinant dans la cuvette (106) de toilettes, et
▪ une face arrière (222) opposée à la face avant (220);
∘ un élément d'attache (510) disposé sur la face arrière (222) du boîtier (204),
- un bras de fixation (600) configuré pour positionner le dispositif d'analyse d'urine (100) entièrement dans la cuvette (106) de toilettes, le bras de fixation (600) comprenant une portion d'attache proximale (710) configurée pour coopérer avec l'élément d'attache (510) du dispositif d'analyse d'urine (100) ;
dans lequel la portion d'attache proximale (710) forme un espace de réception (750) pour le boîtier (204), dans lequel l'espace de réception s'étend depuis la face arrière jusqu'au-delà d'un bord (550) de la face arrière.

2. Ensemble selon la revendication 1, dans lequel la portion d'attache proximale (710) s'étend notamment entre une première extrémité (560) et une deuxième extrémité (570), dans lequel la première extrémité (560) est au niveau de l'élément d'attache (510) sur la face arrière (222) et la deuxième extrémité (570) est au-delà du bord (550) de la face arrière (222).

3. Ensemble selon la revendication 2, dans lequel la première extrémité (560) s'étend jusqu'à la face avant (220) du boîtier (204).

4. Ensemble selon la revendication 2 ou 3, dans lequel la deuxième extrémité (570) comprend une butée (610) configurée pour bloquer un mouvement du dispositif d'analyse d'urine (100) autrement autorisé par l'élément de fixation (600).

5. Ensemble selon l'une quelconques des revendications précédentes, dans lequel l'espace de réception (750) est de forme complémentaire au boîtier (204), avec notamment un jeu fonctionnel.

6. Ensemble selon l'une quelconques des revendications précédentes, dans lequel l'élément d'attache (510) est disposé dans la partie supérieure de la face arrière (222), par exemple entre 15% et 25% en partant du bord (550) de la face arrière (222).

7. Ensemble selon l'une quelconques des revendications précédentes, dans lequel le bras de fixation (600) comprend en outre une portion d'attache distale (720) configurée pour coopérer avec la cuvette (106) de toilettes.

8. Ensemble selon la revendication 7, dans lequel le bras de fixation (600) comprend en outre une portion intermédiaire (730) arrangée entre la portion d'attache proximale (710) et la portion d'attache distale (720).

9. Ensemble selon les revendications 2 et 8, dans lequel la portion d'attache proximale (710) est reliée à la portion intermédiaire (730) au niveau de la deuxième extrémité (570).

10. Ensemble selon la revendication 8 ou 9, dans lequel le bras de fixation (600) présente un changement de direction entre la portion intermédiaire (730) et la portion d'attache proximale (710).

11. Ensemble selon l'une quelconque des revendications 8 à 10, dans lequel la portion intermédiaire (730) et la portion d'attache proximale (710) forment entre elles au niveau de leur contact un angle (α) inférieure à 90°, notamment inférieure à 60°.

12. Ensemble selon l'une quelconque des revendications 8 à 11, dans lequel la portion intermédiaire (730) s'étend dans le prolongement du boîtier (204) dans le plan médian (XZ) du boîtier (204).

13. Ensemble selon l'une quelconque des revendications 8 à 12, dans lequel la portion intermédiaire (730) présente une forme convexe.

14. Ensemble selon l'une quelconque des revendications précédentes, dans lequel la face arrière (222) comprend au moins un écarteur (520) faisant saillie de la face arrière (222) et configuré pour être en contact avec la cuvette des toilettes (106).
